# EUROPÄISCHE PATENTSCHRIFT

(11) **EP 3 116 560 B1**
(45) Veröffentlichungstag und Bekanntmachung des Hinweises auf die Patenterteilung: **04.09.2019**
(21) Anmeldenummer: 15715686.0
(22) Anmeldetag: 13.03.2015
(51) Int. Cl.: A61M 1/14, A61M 1/16

(54) **BLUTBEHANDLUNGSKASSETTE MIT FOLIENVENTIL UND UNELASTISCHEM ABSTANDHALTER SOWIE BLUTBEHANDLUNGSVORRICHTUNG**
BLOOD TREATMENT CARTRIDGE WITH A FILM VALVE AND NONELASTIC SPACER, AND BLOOD TREATMENT DEVICE
CASSETTE DE TRAITEMENT SANGUIN DOTÉE D'UNE VALVE À FEUILLE ET D'UN ÉLÉMENT D'ESPACEMENT NON ÉLASTIQUE ET DISPOSITIF DE TRAITEMENT SANGUIN

(30) Priorität: 14.03.2014 DE 102014103491
(43) Veröffentlichungstag der Anmeldung: 18.01.2017
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2015/055295
(87) Internationale Veröffentlichungsnummer: WO 2015/136074

(56) Entgegenhaltungen:
- WO-A1-2010/121740
- WO-A1-2010/121819
- WO-A1-2014/035471
- US-A- 4 842 584
- US-A1- 2009 215 602

## Beschreibung

Die vorliegende Erfindung betrifft eine Blutbehandlungskassette gemäß dem Oberbegriff des Anspruchs 1 und eine Blutbehandlungsvorrichtung gemäß dem Oberbegriff des Anspruchs 14.

Einmalteilsysteme werden in der Medizin- oder Labortechnik zunehmend als kompakte medizinische Funktionsvorrichtungen wie Kassettensysteme oder Blutbehandlungskassetten ausgeführt, in denen Flüssigkeiten und Gase, insbesondere medizinische Flüssigkeiten und Blut, in Kanälen und Kammern geführt werden. Sind sie zum einmaligen Gebrauch vorgesehen, so spricht man von Kassettendisposables oder Einwegkassetten.

Zumeist handelt es sich hierbei um Hartteil-Folie-Kassetten. Das Hartteil besteht regelmäßig aus einem Spritzgußwerkstoff wie beispielweise PE, PP, PA, ABS, PMMA, PC oder PVC. In ihm sind beispielsweise Schlauchanschlüsse, Konnektoren, Kammern, Kanäle und Zentriereinrichtungen ausgestaltet. Die Kammern und Kanäle sind in der Regel als fluidführende halboffene Strukturen ausgestaltet. Eine Folie aus zum Hartteil kompatiblen Werkstoffen (mit dem Hartteil verschweißbar oder verklebbar) verschließt die halboffenen Strukturen und komplettiert sie zu vollwertigen Kammern und Kanälen. Die Folie kann beispielweise nur an einem äußeren umlaufenden Rand mit der Blutbehandlungskassette verschweißt oder verklebt sein. Ebenfalls gibt es Ausführungen, bei denen die Berandungen der Kammern und Kanäle, die sog. Kanalrandstege, streifenförmig oder flächig mit der Folie verschweißt oder verklebt sind. Auf diese Weise entstehen Blutbehandlungskassetten, die vor dem Einbau in eine Behandlungsmaschine und nach dem Ausbau bereits eine definierte Fluidführung vorgeben.

Bestimmte Bereiche des Hartteils und der Folie werden häufig bewusst nicht miteinander verschweißt oder verklebt. Diese Bereiche können als Folienventile zwischen verschiedenen fluidführenden Bereichen genutzt werden. Zu diesem Zweck wird die Blutbehandlungskassette beim Einsetzen in die Blutbehandlungsvorrichtung zwischen eine Tür und eine Aktor-Sensor-Einheit der Blutbehandlungsvorrichtung eingebaut und anschließend die Tür durch ihr Schließen in eine sog. Verpress-Stellung gebracht, bei der die Folie gegen das Hartteil gepresst und die Blutbehandlungskassette mit der Folie räumlich definiert an die Aktor-Sensor-Matte der Aktor-Sensor-Einheit angekoppelt werden. In die Aktor-Sensor-Matte und Aktor-Sensor-Platte (oder -einheit) integrierte Aktoren (hierin auch als Aktuatoren bezeichnet) können Bewegungen durch die Folie hindurch ausüben, wodurch beispielsweise Pump- oder Ventil-Funktionen realisierbar sind. Mittels wenigstens eines, optional in der Aktor-Sensor-Platte vorgesehenen Sensors können beispielsweise Eigenschaften von Fluiden, welche die Blutbehandlungskassette durchströmen, gemessen werden.

Insbesondere bei Blutbehandlungskassetten mit kanalrandbündig aufgeschweißten Folien und Folienventilen kann es allerdings zu herstelltechnischen oder verfahrenstechnischen Problemen kommen.

Aus der WO 2014/035471 A1 ist eine Spring-Open-Folie für eine blutführende Kassette bekannt.

In der WO 2010/121819 A1 sind eine externe Funktionseinrichtung, eine Blutbehandlungsvorrichtung zum Aufnehmen derselben sowie Verfahren offenbart.

Aus der WO 2010/121740 A1 sind eine Aufnahmeeinrichtung zum Aufnehmen von medizinischen Fluiden sowie eine externe Funktionseinrichtung und eine medizinische Behandlungsvorrichtung bekannt.

In der US 4 842584 A ist eine Einweg-Flüssigkeitsinfusionspumpenkammerkassette und deren Antriebsmechanismus offenbart.

Eine erfindungsgemäße Aufgabe kann darin bestehen, eine weitere Blutbehandlungskassette vorzuschlagen. Ferner soll eine Blutbehandlungsvorrichtung zum Verwenden der Blutbehandlungskassette angegeben werden.

Die Aufgabe kann gelöst werden durch eine Blutbehandlungskassette mit den Merkmalen des Anspruchs 1, ferner durch eine Blutbehandlungsvorrichtung mit den Merkmalen des Anspruchs 14.

Erfindungsgemäß wird somit eine Blutbehandlungskassette mit einem als Hartteil ausgestalteten Kassettenkörper und einer Folie vorgeschlagen. Dabei ist die Folie mit dem Hartteil, beispielsweise durch Schweißen oder Kleben, verbunden und das Hartteil ist zumindest abschnittsweise gegenüber einem Äußeren durch die Folie be- oder abdeckt, so dass aus Hartteil und Folie gemeinsam gebildete Kanäle oder Kammern oder Teile hiervon ausgestaltet werden. Ferner weist das Hartteil wenigstens einen Ventilsitz oder -abschnitt eines Ventils auf, hierin auch als Ventilgrund bezeichnet.

Das Ventil ist ausgestaltet, um eine erste und eine hiervon verschiedene, zweite Stellung oder Ventilstellung einzunehmen. Dabei ist die erste Stellung eine geöffnete Stellung, in welcher das Ventil, insbesondere zum Gassterilisieren von Abschnitten des Hartteils, geöffnet ist. In der ersten Stellung berühren sich der Ventilgrund und ein Abschnitt der Folie, welcher im Gebrauch der Behandlungskassette über dem Ventilgrund angeordnet ist oder zu liegen kommt, nicht. Das Ventil ist konfiguriert und vorgesehen, um bei Aufbringen einer - insbesondere in Richtung auf den Ventilgrund hin und mit Hilfe und/oder unter Wirkung einer auf den über dem Ventilgrund angeordneten Abschnitt der Folie wirkenden - Kraft in die zweite, geschlossene Stellung überzugehen. In der zweiten Stellung berühren sich der Ventilgrund und der Abschnitt der Folie, beispielsweise direkt oder indirekt. In der zweiten Stellung ist das Ventil geschlossen.

Des Weiteren berühren sich das Hartteil und die Folie in einer Hauptberührungsebene und sind miteinander in der Hauptberührungsebene entlang von Kanalstegen verbunden. Letztere säumen oder begrenzen Kammern oder Kanäle des Hartteils. Die Blutbehandlungskassette weist im Bereich des Ventils wenigstens einen mit dem Hartteil integral hergestellten Höcker auf. Dabei ist der Höcker angeordnet, um im unbelasteten oder unverpressten Nichtgebrauchszustand der Blutbehandlungskassette ein Verbleiben des Ventils in der ersten, geöffneten Stellung zu gewährleisten.

Ferner wird eine Blutbehandlungsvorrichtung vorgeschlagen, welche mit einer erfindungsgemäßen Blutbehandlungskassette verbunden ist und eine Aktor-Sensor-Platte mit wenigstens einem Aktuator aufweist. Letzterer ist für eine Interaktion zwischen Aktoren und/oder Sensoren der Blutbehandlungsvorrichtung mit Vorrichtungen der Blutbehandlungskassette vorgesehen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.
Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale in beliebiger Kombination aufweisen.

In gewissen erfindungsgemäßen, beispielhaften Ausführungsformen ist das Ventil ausgestaltet, um mittels Druckaufbringung eines Aktors oder Aktuators einer Blutbehandlungsvorrichtung auf das Ventil, zu deren Betrieb die Blutbehandlungskassette bestimmungsgemäß mit der Blutbehandlungsvorrichtung verbunden wird, von der ersten Stellung in die zweite Stellung überführbar zu sein oder übergeführt zu werden.

In bestimmten erfindungsgemäßen, beispielhaften Ausführungsformen ist das Ventil als Folien- oder Phantomventil ausgestaltet.

In einigen erfindungsgemäßen, beispielhaften Ausführungsformen steht der Höcker über die Hauptberührungsebene hinaus in Richtung weg vom Hartteil über.

In manchen erfindungsgemäßen, beispielhaften Ausführungsformen ragt der Höcker um das 2- bis 4-fache der Dicke oder Stärke der Folie über den Ventilgrund hinaus.

In gewissen erfindungsgemäßen, beispielhaften Ausführungsformen ist zwischen dem Ventilgrund und dem nächstgelegenen Abschnitt des Höckers in einer Ebene parallel zur Haupterstreckungsebene der Folie ein Abstand zwischen dem 6- bis 12-fachen der Dicke oder Stärke der Folie vorgesehen. In bestimmten erfindungsgemäßen, beispielhaften Ausführungsformen weist ein Abschnitt des Höckers, vorzugsweise der Höckerstirnfläche, also jenes Abschnitts, mit welchem der Höcker die Folie berührt oder berühren kann, einen Durchmesser auf. Dieser beträgt das 5- bis 12-fache der Dicke oder Stärke der Folie.

In einigen erfindungsgemäßen, beispielhaften Ausführungsformen ist der Höcker kein Abschnitt der Kanalstege, welche Kammern oder Kanäle des Hartteils säumen oder begrenzen.

Der Höcker ist in bestimmten erfindungsgemäßen, beispielhaften Ausführungsformen bestenfalls in einem Kanal oder in einer Kammer angeordnet. Er ist allerdings nicht Teil des strömungsleitenden Randes von Kanal oder Kammer.

In manchen erfindungsgemäßen, beispielhaften Ausführungsformen ist zwischen dem Höcker und benachbarten Kanalstegen ein Abstand vorgesehen. Dieser entspricht dem 3- bis 8-fachen des Durchmessers des Höckers, insbesondere in einem Bereich, in dem der Höcker die Folie berührt.

In gewissen erfindungsgemäßen, beispielhaften Ausführungsformen ist der Ventilgrund auf Höhe der Haupterstreckungsebene der Folie angeordnet.

Erfindungsgemäß ist der Ventilgrund in Längsrichtung hiervon konkav oder konvex ausgestaltet.

In manchen erfindungsgemäßen, beispielhaften Ausführungsformen beträgt eine Dellentiefe (T) das 2- bis 4-fache der Dicke oder Stärke der Folie.

In gewissen erfindungsgemäßen, beispielhaften Ausführungsformen weist der Ventilgrund eine Länge auf, welche dem 10- bis 30-fachen der Dicke oder Stärke der Folie entspricht.

In bestimmten erfindungsgemäßen, beispielhaften Ausführungsformen entspricht die Dellentiefe des Ventilgrunds dem 1- bis 3-fachen der Dicke oder Stärke der Folie. Alternativ kann der Ventilgrund hinter benachbarten Kanalrandstegen um das 1- bis 3-fache der Dicke oder Stärke der Folie in Richtung Inneres der Blutbehandlungskassette zurückgesetzt sein.

In einigen erfindungsgemäßen, beispielhaften Ausführungsformen weist die Aktor-Sensor-Platte eine Aktor-Sensor-Matte auf, welche direkt oder indirekt in Kontakt mit der Blutbehandlungskassette steht. Dabei ist die Aktor-Sensor-Matte in einem Abschnitt, welcher in Kontakt mit dem Höcker der Blutbehandlungskassette steht, dünner ist als benachbarte Abschnitte der Aktor-Sensor-Matte. In den dünneren Abschnitt fährt im Gebrach der Blutbehandlungskassette ein Aktor ein.

In manchen erfindungsgemäßen, beispielhaften Ausführungsformen hat die Aktor-Sensor-Matte im Abschnitt eine Delle oder eine Aussparung. Die Aktor-Sensor-Matte ist derart ausgestaltet, dass sie im Bereich ihrer Delle oder Aussparung nicht mit der Aktor-Sensor-Platte in Berührung steht.

In gewissen erfindungsgemäßen, beispielhaften Ausführungsformen steht die Aktor-Sensor-Matte direkt oder indirekt in Kontakt mit der Blutbehandlungskassette. Dabei weist die Aktor-Sensor-Platte und/oder die Aktor-Sensor-Matte in einem Abschnitt, welcher in Kontakt mit dem Ventilgrund des Ventils steht, einen starren, jedenfalls nicht mittels der Blutbehandlungsvorrichtung in seiner Höhe, Ausrichtung oder Anordnung veränderbaren Höcker. Der Höcker kann eine Verstärkung des Mattenmaterials sein.

In bestimmten erfindungsgemäßen, beispielhaften Ausführungsformen kann die Verstärkung eine Ausbeulung sein und das 1- bis 3- fache über die Haupterstreckungsebene der Aktor-Sensor-Matte in Richtung Blutbehandlungskassette überstehen.

In bestimmten erfindungsgemäßen, beispielhaften Ausführungsformen weist der Ventilgrund keine Hinterschnitte auf.

Die hierin beschriebenen Folienventile werden im Zusammenhang mit der vorliegenden Erfindung auch Phantomventile genannt, da sie im geschlossenen Zustand aus der Sicht der betroffenen Kanäle keine Veränderung des Strömungsraumes im Vergleich zu Kanalstellen oder Kammern ohne Folienventil darstellen. Sie werden mit Blick auf den Strömungsraum - gleich einem Phantom - nicht wahrgenommen.

In bestimmten erfindungsgemäßen, beispielhaften Ausführungsformen beult der Höcker, hierin auch als Beabstandungsvorrichtung bezeichnet, über ihm liegenden Abschnitt der Folie lokal durch direkten oder indirekten Kontakt mit dieser aus. Auf diese Weise schafft er einen Abstand des betreffenden Folienabschnitts zu einem benachbarten Kanalsteg und/oder dem Ventilgrund. Der Höcker ragt dabei in manchen erfindungsgemäßen Ausführungsformen über die allgemeine Ebene der flachen Folie hinaus, also über jene Ebene, in welcher die Folie im Wesentlichen plan auf dem Hartteil aufliegt, beispielsweise über die Verklebe- oder Verschweißebene, hierin auch als Haupterstreckungsebene der Verbindung bezeichnet, zu dieser parallel oder mit dieser zusammenfallend. Dies kann vorteilhaft einen Sicherheitsabstand schaffen, welcher ein zuverlässiges Durchströmen des offen gehaltenen Ventils erlaubt.

In einigen erfindungsgemäßen, beispielhaften Ausführungsformen ist der Höcker angeordnet, um die Folie auch im Falle eines Unterdrucks lokal in der allgemeinen Ebene der flachen Folie zu halten. Da der Höcker dabei die Folie im Bereich des Ventils nicht über die allgemeine Ebene hinaus anhebt, kann hierbei vorteilhaft eine ungewollte und möglicherweise irreversible Dehnung des Folienabschnitts verhindert werden.

In bestimmten erfindungsgemäßen, beispielhaften Ausführungsformen ist der Höcker federnd und/oder nachgiebig im Hartteil angeordnet.

In einigen erfindungsgemäßen, beispielhaften Ausführungsformen weist die Blutbehandlungskassette nicht einen Höcker, sondern mehrere Höcker auf, für die das hierin zum Höcker ebenfalls gelten kann.

In bestimmten erfindungsgemäßen, beispielhaften Ausführungsformen sind mehrere Höcker (hierin allgemein auch als Erhebungen zu bezeichnen) in wenigstens einer Reihe parallel zum Verlauf des Dichtsitz-Steges oder Ventilgrunds angeordnet. Diese Anordnung kann die Folie vorteilhaft auch dann auf aus strömungstechnischer Sicht ausreichendem Abstand vom Dichtsitz-Steg oder vom Ventilgrund halten, wenn die Höcker ausgestaltet sind, um nicht oder nur geringfügig über Kanalrandstege, mit welchen die Folie verbunden ist, überzustehen. Der Strömungsquerschnitt wird über die Länge des mittels Abstandhalters erzeugten Durchlasses zwischen Ventilgrund und Folie erzeugt.

In bestimmten erfindungsgemäßen, beispielhaften Ausführungsformen sind der oder die Höcker von Drainagestrukturen durchzogen oder hiermit versehen. Letztere verringern eine Kontaktfläche zwischen Höcker und Folie während der Sterilisation und können hierdurch vorteilhaft zu einer erhöhten Wirksamkeit des Sterilisationsprozesses beitragen.

In manchen erfindungsgemäßen, beispielhaften Ausführungsformen sind die Höcker auf einem oder mehreren Federelementen angeordnet.

In manchen erfindungsgemäßen, beispielhaften Ausführungsformen ist der Höcker angeordnet, um einen Abstand des Folienabschnitts vom Ventilgrund sicherzustellen, welcher einen Gas- oder Dampfaustausch über das Ventil hinweg, also zwischen Folie und Ventilgrund, zulässt.

In bestimmten erfindungsgemäßen, beispielhaften Ausführungsformen wirkt der Höcker von einem Inneren der Blutbehandlungskassette, also einem Raum zwischen Hartteil und Folie, auf die Folie ein.

In manchen erfindungsgemäßen, beispielhaften Ausführungsformen weist die Blutbehandlungsvorrichtung einen unterteilten oder lamellierten Anpressstempel als Aktor auf. Die Unterteilung oder Lamellierung erlaubt es dem Aktor auf Krümmungen des Ventilgrunds einzugehen.

In bestimmten erfindungsgemäßen, beispielhaften Ausführungsformen weist der unterteilte oder lamellierte Anpressstempel eine reibungsfreie Festkörpergelenk-Lagerung und/oder eine Federunterstützung auf.

In einigen erfindungsgemäßen, beispielhaften Ausführungsformen weist die Blutbehandlungsvorrichtung anstelle des oder ergänzend zum lamellierten Anpressstempel mit vorzugsweise reibungsfreier Festkörpergelenk-Lagerung und Federunterstützung andere Toleranzausgleichsvorrichtungen in der Aktor-Sensor-Einheit auf. Zu ihnen zählen strukturierte oder inseitig gehöckerte Aktor-Sensor-Matten, strukturierte oder gehöckerte Aktor-Sensor-Platten, zwischengeschaltete Schaumstoffe und Druck-Kissen, strukturierte zwischengeschaltete Elastomerkörper und dergleichen.

In manchen erfindungsgemäßen, beispielhaften Ausführungsformen sind die hierin beschriebenen Folienventile solche, welche auch während des Gebrauchs der Blutbehandlungskassette eine Funktion einnehmen. In anderen beispielhaften erfindungsgemäßen Ausführungsformen dienen die Folienventile allein als Ventile, mit denen vor Gebrauch der Blutbehandlungskassette ein Übertritt von Sterilisationsmittel zwischen Kammer und Kanal/Kammer sichergestellt werden soll. In diesen Ausführungsformen bleibt das Folienventil nach Beginn der Blutbehandlungssitzung mittels Aktuators verschlossen.

Einige oder alle erfindungsgemäßen Ausführungsformen können einen oder mehrere der oben oder im Folgenden genannten Vorteile aufweisen.

Beim Sterilisieren der Blutbehandlungskassetten mit Gasen oder mit Dampf finden zum Gasaustausch in der Regel mehrere Wechsel zwischen Vakuum- und Überdruck-Phasen statt. Dabei wirken die verbleibenden, nicht verschweißten oder verklebten Dichtsitzbereiche der Ventile als Strömungswiderstände. Bei der Umschaltung von Vakuum auf Überdruck wirken sie gelegentlich und unbeabsichtigt sogar als Rückschlagventil, wenn sich die Folie wegen des in der Blutbehandlungskassette erst verzögert hergestellten Druckausgleichs in Richtung auf die Kavitäten und Kammern der Blutbehandlungskassette verformt. Dabei schließen sich die Folienventile möglicherweise und können dadurch den Zutritt von Sterilisationsgasen in einzelne Kanäle oder Kammern be- oder gar verhindern, weshalb es zu unzureichender Sterilisierung kommen kann. Zum Lösen dieses Problems musste bislang auf unwirtschaftliche Sterilisationsverfahren zurückgegriffen werden. Diese Nachteile können mittels der erfindungsgemäßen Blutkassette verringert oder vermieden werden, denn ihre Folienventile bleiben Dank des Höckers beim Sterilisieren selbst bei Anlegen von Unterdruck offen.

Aufgrund des erfindungsgemäß vorgesehenen Höckers, welcher die Folie über den Folienventilen, an welchen diese nicht mit dem Hartteil verschweißt sind, unter Spannung hält, können die aufgrund der Wechsel von Vakuum zu Überdruck und umgekehrt auftretenden mechanischen Beanspruchungen der nicht verschweißten Folieabschnitte verhindert oder zumindest vermindert werden. Dies kann auch einem sich aufgrund der mechanischen Beanspruchungen ergebenden Durchhängen der Folien entgegenwirken.

Erfindungsgemäß wird somit eine technische Lösung vorgeschlagen, die es vorteilhaft erlaubt, die Folie an einer Stelle oder an mehreren Stellen der Berandungen der Kammern und Kanäle vom Hartteil zu beabstanden, so dass analog zur Funktionsweise der bereits beschriebenen Phantomventile eine fluidische Verbindung zwischen Kammern und Kanälen zustande kommt und damit der Transport der Sterilisationsgase verbessert und die bei den Druckwechseln entstehenden Folienbelastungen reduziert werden.

Zu den weiteren Vorteilen zählt, dass im Zusammenhang mit einer aufgrund der Folien-Herstelltoleranzen bereits leicht delligen Folie dennoch ausgeschlossen werden kann, dass die Folie im Bereich der Ventildichtsitzkante bereits vor der Gassterilisationsbehandlung flächigen Kontakt mit derselbigen aufnehmen kann und vielmehr lokal beabstandet bleibt, bis die Blutbehandlungskassette das Sterilisationsverfahren absolviert hat.

Die Toleranzausgleichsvorrichtungen mittels der Aktor-Sensor-Einheit können dabei vorteilhaft die Dichtwirkung von flachen oder gedellten Folienventilen aufrechterhalten und sind robust gegenüber Maßtoleranzen der Blutbehandlungskassette, der Blutbehandlungsvorrichtung, oder die Ausrichtung zwischen Blutbehandlungskassette, der Blutbehandlungsvorrichtung betreffend.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil vereinfachten Figuren gilt:
- Fig. 1: zeigt eine Seitenansicht einer an ihrer Vorderseite mit einer Abdeckungseinrichtung versehenen erfindungsgemäßen Blutbehandlungskassette gemäß einer bevorzugten Ausführungsform;
- Fig. 2: zeigt die externe Funktionseinrichtung der Fig. 1 mit nach zerstörendem Aufschneiden aufgeklappter Abdeckungseinrichtung;
- Fig. 3: zeigt die Blutbehandlungskassette aus Fig. 1 und Fig. 2 von ihrer Rückseite;
- Fig. 4: zeigt in Draufsicht einen Ausschnitt eines Hartteils einer erfindungsgemäßen Blutbehandlungskassette;
- Fig. 5: zeigt den Blick auf die in Fig. 4 dargestellte Blutbehandlungskassette in einer ersten Ausführungsform in Richtung der Pfeile A der Fig. 4;
- Fig. 6: zeigt den Blick auf die in Fig. 4 dargestellte Blutbehandlungskassette in einer zweiten Ausführungsform gemäß der vorliegenden Erfindung, wiederum in Richtung der Pfeile A der Fig. 4;
- Fig. 7: zeigt ein Ventil der erfindungsgemäßen Blutbehandlungskassette;
- Fig. 8: zeigt die Blutbehandlungskassette der Fig. 5, eingelegt in eine Ausführungsform der Blutbehandlungsvorrichtung und von dieser zwischen Tür und Aktor-Sensor-Matte verpresst; und
- Fig. 9: zeigt die Blutbehandlungskassette der Fig. 6, eingelegt in eine Ausführungsform der Blutbehandlungsvorrichtung und von dieser zwischen Tür und Aktor-Sensor-Matte verpresst.

Die normalen Pfeile in den Figuren zeigen die Richtung des Blutstroms an. Die Blockpfeile zeigen jeweils die Richtung des Substituatstroms an.

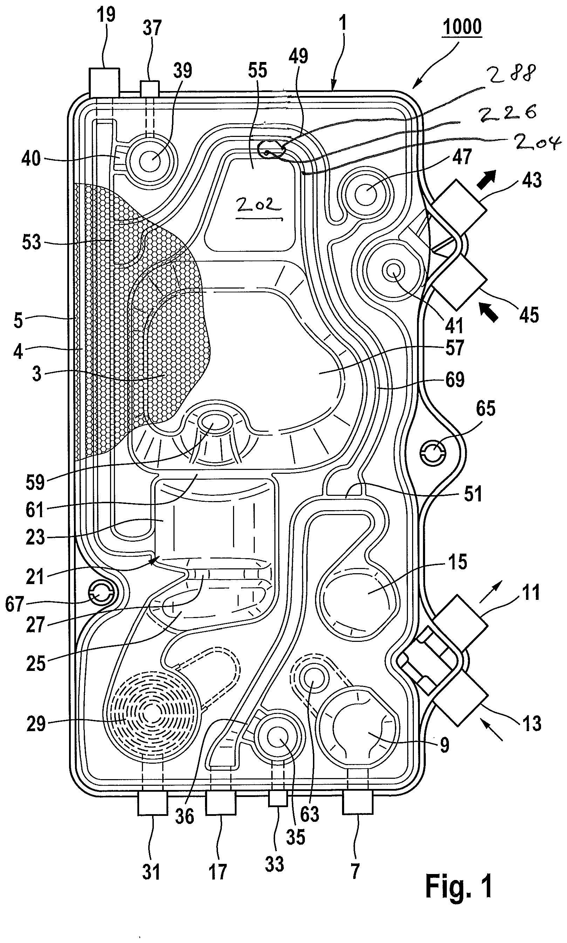

**Fig. 1** zeigt eine Seitenansicht einer erfindungsgemäßen Blutbehandlungskassette 1000, welche an der Oberfläche, auf die in Fig. 1 geblickt wird, mit einer Abdeckungseinrichtung versehen ist.

Die erfindungsgemäße Blutbehandlungskassette 1000 wird im Folgenden auch kurz als Kassette 1000 bezeichnet.

Die Kassette 1000 weist ein Hartteil 1 auf. Wie in Fig. 1 exemplarisch gezeigt, weist das Hartteil 1 Kammern, Kanäle und Ventile auf. Wie in Fig. 1 exemplarisch weiter gezeigt ist, sind die Kammern, Kanäle und Ventile in das Hartteil 1 integriert bzw. zumindest teilweise vom Hartteil 1 ausgestaltet.

Die Kassette 1000 der Fig. 1 ist an ihrer Vorderseite mit einer Abdeckungseinrichtung, hier beispielsweise einer Folie 3, versehen. Die Abdeckungseinrichtung kann eben, d. h. plan, auf das Hartteil 1 aufgeschweißt sein.

Eine Ausgestaltung mit einer dreidimensionalen Ausführung der Schweiß- und Dichtkontur ist erfindungsgemäß ebenfalls möglich.

Die Abdeckungseinrichtung kann die Kammern und/oder Kanäle des Hartteils 1 der Kassette 1000 verschließen, und zwar gegenüber einer dem Hartteil 1 abgewandten Seite der Abdeckungseinrichtung und/oder gegenüber der Atmosphäre.

Wie in Fig. 1 zu erkennen ist, liegt die Folie an einem umlaufenden Dichtsteg 4 auf dem Hartteil 1 der Kassette 1000 auf. Die Folie 3 ist an einer umlaufenden Schweißnaht 5 mit dem Hartteil 1 der Kassette 1000 verschweißt.

Der umlaufende Dichtsteg kann alternativ freiliegend ausgeführt werden.

Die Folie 3 kann an weiteren lokalen Verschweißungen (nicht gezeigt) mit dem Hartteil 1 der Kassette 1000 verbunden sein. Diese können ebenfalls umlaufend, also geschlossen im Sinne einer abschließenden Begrenzung ähnlich einem Ring, und/oder punktförmig sein.

Die Folie 3 kann an lokalen Stellen punkt- oder linienförmig mit dem Hartteil der Kassette 1000 verbunden, z. B. verschweißt, sein, insbesondere an den Randzonen der flüssigkeitsführenden Kanäle.

Die Folie 3 kann durch Laser-Schweißen mit dem Hartteil der Kassette 1000 verbunden werden. Dabei ist es vorteilhaft, wenn der lokale Hitzeeintrag unter Verwendung einer lichtabsorbierenden Komponente erfolgt. Die lichtabsorbierende Komponente kann Bestandteil des Materials der Folie und/oder des Hartteils sein oder eine Schicht, die zwischen Folie und Hartteil oder über der Folie angeordnet ist. Die Schicht kann eine Folienschicht sein.

Die Kassette 1000 kann wenigstens mit der in Fig. 1 gezeigten Vorderseite an eine Blutbehandlungsvorrichtung (in Fig. 1 nicht gezeigt) angekoppelt werden. Eine beispielhafte Vorgehensweise zum geeigneten Ankoppeln einer Kassette 1 an eine Ankoppelfläche einer Blutbehandlungsvorrichtung ist in den Patentanmeldungen 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, und 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die ebenfalls am 10. März 2009 beim DPMA eingereicht wurde, beschrieben.

Die Kassette 1000 kann mit der Ebene der Folie 3 - oder über diese - an eine Ankoppelfläche der Blutbehandlungsvorrichtung angekoppelt werden. Die Ankoppelfläche kann vorzugsweise dreidimensional ausgeführt sein.

Die Ankoppelfläche der Blutbehandlungsvorrichtung kann zum Beispiel an einem in Fig. 1 oberen Abschnitt hiervon um 8° gegen eine in Fig. 1 von oben nach unten verlaufende Senkrechte nach hinten (in der Fig. 1 die sich vom Betrachter in die Zeichenebene hinein erstreckende Richtung) geneigt sein.

Die Kassette 1000 weist einen arteriellen Patientenanschluss 7 auf.

Die Kassette 1000 weist eine arterielle Druckmesskammer 9 auf. Diese kann entsprechende Sensoren aufweisen. Die Sensoren können bevorzugt über eine Verkabelung Signale übermitteln. Die Sensoren können aber auch so ausgeführt sein, dass sie kabellos Signale übermitteln.

Die Kassette 1000 weist einen Konnektor 11 für den Blutaustritt aus der Kassette 1000 sowie einen Konnektor 13 für den Bluteintritt in die Kassette 1000 auf.

Die beiden Konnektoren 11 und 13 sind mit einem Pumpschlauchsegment oder -set einer Blutpumpe verbindbar.

Die Kassette 1000 weist ferner eine Kammer 15 mit einer Druckmessstelle zur Druckmessung im extrakorporalen Blutkreislauf vor dem Dialysator ("Prä-Filter") bzw. nach der Pumpe ("Post-Pumpe") auf.

An der Kammer 15 kann durch die Folie 3 hindurch bzw. über die Folie 3 der Druck im extrakorporalen Kreislauf vor dem Dialysator gemessen werden.

Die Kassette 1000 weist eine arterielle Filterleitung 17 sowie eine venöse Filterleitung 19 auf.

Das Innere der Kassette 1000 weist eine venöse Blutkammer 21 auf. Die venöse Blutkammer 21 ist einen oberen Raum 23 und einen unteren Raum 25 unterteilt.

Der obere Raum 23 der venösen Blutkammer 21 kann eine seitlich tangentiale Bluteinströmung zulassen. Dabei kann Blut seitlich durch den Einlass (der linken Seite in Fig. 1) in den oberen Raum 23 einströmen und sich tangential zu den Wänden des oberen Raums 23 ausbreiten. Eine seitlich tangentiale Bluteinströmung kann eine Zone mit im Wesentlichen oder vollständig stabiler Rotationsströmung des Bluts in dem oberen Raum 23 der venösen Blutkammer 21 erzeugen.

Der untere Raum 25 der venösen Blutkammer 21 kann eine Beruhigungszone für die Blutströmung darstellen. Es ist möglich, dass in einer derartigen Beruhigungszone im Wesentlichen keine oder überhaupt keine Rotationsströmung des Bluts vorliegt.

Die venöse Blutkammer 21 ist durch eine Querschnittsverjüngung 27 des Hartteils 1 der Kassette 1000 in den oberen Raum 23 und den unteren Raum 25 aufgeteilt. Die Querschnittsverjüngung 27 reduziert den Querschnitt der venösen Blutkammer 21 derart in seiner Breite und Tiefe, dass sich eine Strömungsschnelle ergibt, nach deren Durchquerung ein die venöse Blutkammer 21 der Kassette 1000 durchströmendes Fluid eine langsamere Strömungsgeschwindigkeit annimmt. Der obere Raum 23 und der untere Raum 25 stehen in Fluidkommunikation.

Durch eine derartige Konstruktion, d.h. einer Aufteilung der venösen Blutkammer 21 in eine Zone mit im Wesentlichen oder vollständig stabiler Rotationsströmung des Bluts und in eine Beruhigungszone für die Blutströmung, kann vorteilhaft eine effiziente Abscheidung für Luft aus dem Blut oder Fluid erreicht werden.

Wandungen des oberen Raums 23 und des unteren Raums 25 der venösen Blutkammer 21 können in geeigneter Weise einer Neigung des oberen Abschnitts der Kassette 1000 in Fig. 1 gegen die Vertikale, beispielsweise einer Neigung des oberen Teils der in Fig. 1 gezeigten Kassette 1000 um 8° nach hinten (in die Zeichenebene hinein), angepasst werden. Sie können in geeigneter Weise derart gerundet ausgestaltet sein, dass sie vorteilhaft eine strömungsoptimierte Berührfläche für Fluide darstellt, welche die venöse Blutkammer 21 durchströmen.

Die Kassette 1000 weist einen Gerinnselfänger 29 auf.

Als Gerinnselfänger kann vorzugsweise ein Gerinnselfänger verwendet werden, wie er in der von der Anmelderin der vorliegenden Erfindung in der Patentanmeldung 10 2009 024 495.6 mit dem Titel *"Gerinnselfänger, externe Funktionseinrichtung, Blutkreislauf sowie Behandlungsvorrichtung",* die am 10. Juni 2009 beim DPMA eingereicht wurde, offenbart wurde.

Am Gerinnselfänger 29 kann durch die Folie 3 hindurch bzw. über die Folie 3 der Druck im extrakorporalen Kreislauf gemessen werden, also insbesondere nach Durchlaufen des Dialysators.

Die Kassette 1000 weist einen venösen Patientenanschluss 31 auf.

Die Kassette 1000 weist eine arterielle
Heparin Zugabestelle 33 auf. Dabei gilt zu beachten, dass die Heparin-Zugabestelle 33 (wie auch eine venöse Heparin Zugabestelle 37) auch zur Zugabe anderer pharmakologischer Wirkstoffe als Heparin, welche nur vorzugsweise Antikoagulantien sind, oder Wirkstoffkombinationen geeignet und vorgesehen sein kann. Dies ist stets auch dann zu beachten, wenn zuvor oder im Folgenden von Heparin in beliebigem Zusammenhang die Rede ist.

Die Kassette 1000 weist ein Rückschlagventil 35 der arteriellen Heparin-Zugabestelle 33 auf.

Beispielhafte Rückschlagventile zum Einsatz als Rückschlagventil 35 der arteriellen Heparin-Zugabestelle 33 sowie als weitere Rückschlagventile der Kassette 1000 sind in der Patentanmeldung 10 2009 024 469.7 der Anmelderin der vorliegenden Erfindung mit dem Titel *"Ventilvorrichtung, Ventileinsatz, externe Funktionseinrichtung, Behandlungsvorrichtung sowie Verfahren",* die am 10. Juni 2009 beim DPMA eingereicht wurde, offenbart.

Die Kassette 1000 weist ein arterielles Heparin-Zugabeventil 36 auf. Mit Hilfe des arteriellen Heparin-Zugabeventils 36 kann die Zugabe von Heparin in die arterielle Filterleitung 17 gesteuert oder geregelt werden.

Das arterielle Heparin-Zugabeventil 36 kann als so genanntes Phantomventil ausgestaltet sein.

Der Begriff "Phantomventil", wie er hierin verwendet wird, bezeichnet ein Element mit einer mittels Aktors erreichbaren Aktor-Fläche (hier beispielsweise eine Aktor-Membran), welches die Funktion eines Ventils übernehmen kann.

Die Aktor-Membran ist unter Aufbringung einer Kraft hierauf, z.B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.

Wenn die Kraft wieder von der Aktor-Membran genommen wird, kann diese in beispielsweise eine Grundposition, z.B. einen nicht gewölbten Zustand, zurückkehren.

Ein Phantomventil zur Verwendung als arterielles Heparin-Zugabeventil 36 sowie weitere Phantomventile der Kassette 1000 können mit oder aus einem Stegabschnitt eines Kanals am Hartteil 1 der Kassette 1000 und einem dem Stegabschnitt an- oder gegenüberliegenden Abschnitt der Folie 3 ausgestaltet werden.

Phantomventile können durch Aktoren der Blutbehandlungsvorrichtung betätigt werden.

Zum Schließen eines Phantomventils kann der Abschnitt der Folie 3 auf den Stegabschnitt gedrückt werden. Zum Öffnen des Phantomventils kann der Abschnitt der Folie 3 wieder vom Stegabschnitt abgehoben werden.

Weitere Beispiele und/oder Ausführungsformen für Phantomventile können der Patentanmeldung 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die am 10. März 2009 von der vorliegenden Anmelderin beim DPMA eingereicht wurde, sowie der DE 100 53 441 A1 und der DE 102 24 750 A1 entnommen werden.

Die Kassette 1000 weist eine venöse Heparin-Zugabestelle 37 auf. Die venöse Heparin-Zugabestelle 37 kann als Luer-Konnektor ausgestaltet sein.

Die Kassette 1000 weist ein Rückschlagventil 39 der venösen Heparin-Zugabestelle 37 auf.

Die Kassette 1000 weist ein venöses Heparin-Zugabeventil 40 auf. Mit Hilfe des venösen Heparin-Zugabeventils 40 kann die Zugabe von Heparin in die venöse Filterleitung 19 gesteuert oder geregelt werden.

Die Kassette 1000 weist eine Substituat-Zugabestelle 41 bzw. einen Substituatkonnektor auf.

Die Substituat-Zugabestelle 41 kann eine Verbindungseinrichtung sein, wie sie in der Patentanmeldung 10 2009 024 575.8 der vorliegenden Anmelderin beschrieben ist, welche mit dem Titel *"Verbindungseinrichtung und Verfahren zum Konnektieren wenigstens zweier fluidführender medizintechnischer Systeme, sowie medizintechnische Vorrichtung"* am 10. Juni 2009 von der vorliegenden Anmelderin beim DPMA eingereicht wurde.

Die Substituat-Zugabestelle 41 kann mit einem Berührschutzelement (nicht gezeigt) versehen sein. Die Substituat-Zugabestelle 41 kann mit einem Tropfschutz (nicht gezeigt) versehen sein. Der Tropfschutz kann durch eine integrierte Verschlusshülse realisiert werden. Der Tropfschutz kann ein Heraustropfen von Resten von Substituat und/oder Blut beim Lösen der Kassette 1000 und anschließendem Entnehmen der Kassette 1000 aus der Blutbehandlungsvorrichtung verhindern.

Der Tropfschutz kann abnehmbar ausgeführt sein. Er kann als Haube oder Deckel ausgestaltet sein.

Die Substituat-Zugabestelle 41 oder ein anderer Abschnitt der Kassette 1000 kann ferner einen Originalitätsschutz bieten, durch welchen der Anwender ohne Mühe oder auf einen Blick erkennt, ob die Kassette 1000 bereits benutzt wurde. Dieser Originalitätsschutz kann mittels des Berührschutzelements, der Verschlusshülse oder einer anderen Struktur realisiert sein. Die entsprechende Struktur kann vorzugsweise ihre Position innerhalb der oder bezogen auf die Kassette 1000 erkennbar verändern. Sie kann vorzugsweise ihre Gestalt verändern.

Zudem kann die Substituat-Zugabestelle 41 oder ein anderer Abschnitt der Kassette 1000 einen Wiederverwendungsschutz bzw. Schutz gegen ein Wiederverwenden bieten. Vorzugsweise durch eine Verschlusshülse wird die Kassette 1000 - vorzugsweise irreversibel - im Hinblick auf den Versuch einer Wiederverwendung unbrauchbar gemacht. Soll die Kassette 1000 trotzdem erneut verwendet werden wollen, so messen Sensoren der Blutbehandlungsvorrichtung nicht die Signalverläufe, die bei Verwenden einer neuen Kassette gemessen werden würden. Dies kann hierauf beruhen, dass keine Flüssigkeit in die Kassette 1000 oder in die Substituat-Zugabestelle 41 gelangen kann oder wenigstens nicht in ausreichender oder üblicher Menge. Die Steuereinheit der Blutbehandlungsvorrichtung kann dies erkennen. Es kann eine Warnung veranlasst werden.

Als Originalitätsschutz oder Wiederverwendungsschutz kann vorzugsweise ein Originalitätsschutz oder ein Wiederverwendungsschutz verwendet werden, wie er in der von der Anmelderin der vorliegenden Erfindung in der Patentanmeldung 10 2009 024 575.8 mit dem Titel *"Verbindungseinrichtung und Verfahren zum Konnektieren wenigstens zweier fluidführender medizintechnischer Systeme, sowie medizintechnische Vorrichtung",* die am 10. Juni 2009 beim DPMA eingereicht wurde, offenbart wurde.

Die Kassette weist einen Konnektor 43 für einen Substituataustritt aus der Kassette 1000 sowie einen Konnektor 45 für einen Substituateintritt in die Kassette 1000 auf.

Die Konnektoren 43 und 45 sind mit einem Pumpschlauchsegment oder -set einer Substituatpumpe verbindbar.

Die Kassette 1000 weist ein Rückschlagventil 47 für Substituatzugabe auf.

Über Betätigung des Rückschlagventils 47 kann Substituat in eine Substituatleitung 49 eingebracht werden.

Die Kassette 1000 weist ein Prädilutions-Zugabeventil 51 auf. Das Prädilutions-Zugabeventil 51 kann als Phantomventil ausgestaltet sein.

Die Kassette 1000 weist ein Postdilutions-Zugabeventil 53 auf. Das Postdilutions-Zugabeventil 53 kann als Phantomventil ausgestaltet sein.

Die Kassette 1000 weist eine Single-Needle-Sterilmembran 55 auf.

Die Kassette 1000 weist eine Single-Needle-Kammer 57 auf. Die Single-Needle-Kammer 57 ist in Fig. 1 oberhalb der venösen Blutkammer 21 angeordnet.

Im Inneren der Single-Needle-Kammer 57 ist ein Blutschwallumleitungselement 59 angeordnet. Das Blutschwallumleitungselement 59 kann dem Abbremsen eines Blutschwalls und/oder dem Auslöschen dessen Impulses dienen.

Eine Verbindung mit einem Inneren der Single-Needle-Kammer 57 kann mittels einer Verbindungseinrichtung vorgesehen sein, wie sie in der von der Anmelderin der vorliegenden Erfindung mit dem Titel *"Einrichtung sowie externe Funktionseinrichtung und Behandlungsvorrichtung zum Behandeln von medizinischen Fluiden"* 10 2009 024 467.0 am 10. Juni 2009 beim DPMA eingereichten Patentanmeldung offenbart wurde.

Die Kassette 1000 weist ein Single-Needle-Blutventil 61 auf. Das Single-Needle-Blutventil 61 kann als Phantomventil ausgestaltet sein.

Die Kassette 1000 weist eine Absaugstelle 63 auf. Die Absaugstelle 63 kann zur Vakuumankopplung der Kassette 1000 an die Blutbehandlungsvorrichtung dienen, wie dies beispielsweise in der Patentanmeldung DE 10 2007 042 964 A1 mit dem Titel *"Vorrichtung und Verfahren zur Behandlung einer medizinischen Flüssigkeit",* die am 10. September 2007 beim DPMA eingereicht wurde, beschrieben ist.

Die Kassette 1000 weist ein primäres Ausrichtungszentrum 65 auf. Das primäre Ausrichtungszentrum 65 kann vorteilhaft zum Ausrichten und/oder Verrasten der Kassette 1000 an der Blutbehandlungsvorrichtung dienen.

Die Kassette 1000 weist eine sekundäre Ausrichtungsstelle 67 auf. Die sekundäre Ausrichtungsstelle 67 kann zum Ausrichten und/oder Verrasten der Kassette 1000 an der Blutbehandlungsvorrichtung dienen.

Die Kassette 1000 ist vor Beginn des Primens mit Gas (z.B. steriler Luft) gefüllt. Beim Primen des extrakorporalen Blutkreislaufs muss diese Gasfüllung verdrängt werden. Eine Blutbehandlungskassette stellt hierbei allgemein eine besondere Herausforderung dar, weil es sowohl aufsteigende als auch fallende Leitungen und zudem Kammern gibt, in denen keine "Luftnester" verbleiben dürfen. Die vorliegende Kassette 1000 weist zu diesem Zweck besondere Konstruktionsmerkmale auf:
Die Kammer 15 zum Messen des arteriellen Drucks ist derart konstruiert, dass die gesamte Luft in ein Pumpschlauchsegment aufsteigen kann. Es gibt hierbei vorteilhaft keine Toträume. Luft, die aus der arteriellen Druckmesskammer in das Pumpschlauchsegment der Blutpumpe selbständig aufsteigt, wird ab dem Eingriffsbereich Blutpumpe (z.B. durch die Rollen einer Rollenpumpe) durch das Pumpschlauchsegment zwangsgefördert. Sobald die Pumpe keinen Einfluss mehr hat (weil z.B. die Rollen in Ausgriff gehen) steigt die Luft selbsttätig in Förderrichtung in die Kassette 1000 auf.

Die venöse Rückführleitung (bzw. ein venöser Abschnitt des extrakorporalen Kreislaufs) ist eine Fallleitung. Ab einem darin herrschenden, gewissen Volumenstrom (z. B. 200 ml/min bei der in Fig. 1 gezeigten Kassette 1000) werden Luftblasen im Blut auch entgegen der Erdbeschleunigung "mitgerissen". Dieser Effekt wird bei den Fallleitungen ausgenutzt. Die Leitungsquerschnitte der Fallleitungen sind so klein ausgelegt, dass durch die Strömungsgeschwindigkeit eine Zwangsförderung der Luftblasen auch entgegen der Erdbeschleunigung funktioniert.

In der venösen Blutkammer 21 sind große Querschnitte vorgesehen, derart, dass aufgrund der dort langsamen bzw. niedrigen Strömungsgeschwindigkeiten Luftblasen verlässlich entgegen der Hauptströmungsrichtung aufsteigen können.

Weitere konstruktive Besonderheiten der Kassette 1000 sind:
Die Phantomventile 40, 51 und 53 sind räumlich derart ausgerichtet, dass Blut (welches eine höhere Dichte als Wasser bzw. Substituat etc. hat) bei Betrieb der Kassette 1000 mit Blut kaum "nach oben" oder "zur Seite" in geöffnete Phantomventile eindringen kann, weil es gegenüber dem leichteren Wasser absinkt. Eine solch vorteilhafte Ausrichtung ist durch die Phantomventile 40, 51, und 53 realisiert. Beim Ventil 36 besteht hingegen kein solches Erfordernis, d.h. dort kommt es auf die Orientierung nicht an.

Der Leitungskanal (Stichkanal) unter dem Rückschlagventil 47 für Substituatzugabe ist aus demselben Grunde aufsteigend konstruiert. Im Falle einer Fehlfunktion der Prä- und/oder Postdilutionsventile 51 und 53 und einer daraus resultierenden Blut-Bypassströmung kann kein Blut in die Substituatleitung 49 aufsteigen. Vielmehr strömt das Blut an der Mündung der entsprechenden Stichleitung vorbei.

Die Neigung der Kassette 1000 beträgt vorzugsweise 5° bis 11°, besonders bevorzugt die bereits genannten 8°.

Das Bezugszeichen 288 bezeichnet ein Phantomventil, welches in einer ersten Stellung einen Einstrom in die Kammer 202 ermöglicht oder in einer zweiten Stellung unterbindet. In seiner einfachsten, von der vorliegenden Erfindung umfassten Ausgestaltung ist ein Phantomventil ein Folienventil, bei welchem ein Fluidweg zwischen Hartteil 1 und darüberliegendem Abschnitt der Folie 3 durch vorübergehendes Anpressen der Folie 3 auf einen Ventilgrund, etwa einen Steg oder anderen Abschnitt des Hartteils 1, unterbunden und nach Aufheben der anpressenden Kraft wieder geöffnet wird.

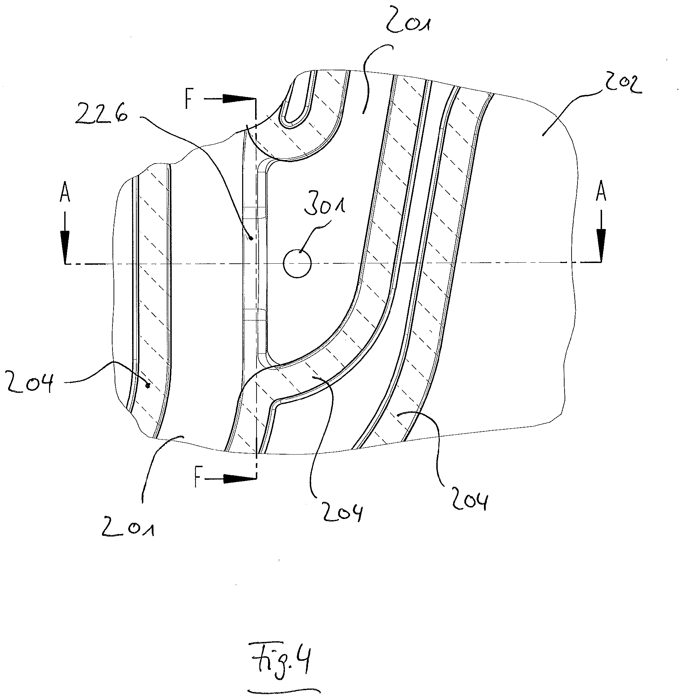

Die Bezugszeichen 202, 204 und 226 werden mit der Beschreibung der Fig. 4 bis 9 erläutert.

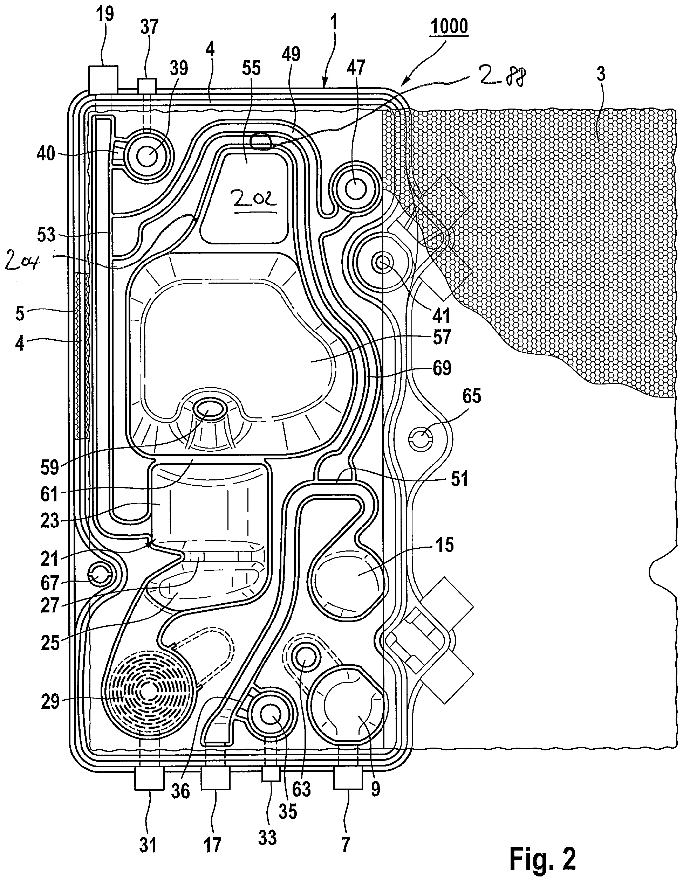

**Fig. 2** zeigt die Kassette 1000 der Fig. 1, wobei die Folie 3 am linken Rand der Kassette 1000 sowie oben und unten zur besseren Illustration zerstörend aufgeschnitten und nach rechts aufgeklappt zu erkennen ist.

Wie in Fig. 2 gezeigt, weist die Folie 3 eine Oberflächenstrukturierung auf.

Fig. 2 zeigt die nach Aufschneiden der Folie 3 detaillierter zu erkennenden Elemente im Inneren der Kassette 1000.

Zur Vermeidung von Wiederholungen wird auf die vorstehend bei der Beschreibung der Fig. 1 erörterten Ausgestaltungen der einzelnen Elemente verwiesen.

Gut zu erkennen ist hierbei, dass die Kassette 1000 einen Dichtsteg 69 aufweist. Der Dichtsteg 69 kann zum Beispiel zum Ausgestalten des Prädilutions-Zugabeventils 51 eingesetzt werden.

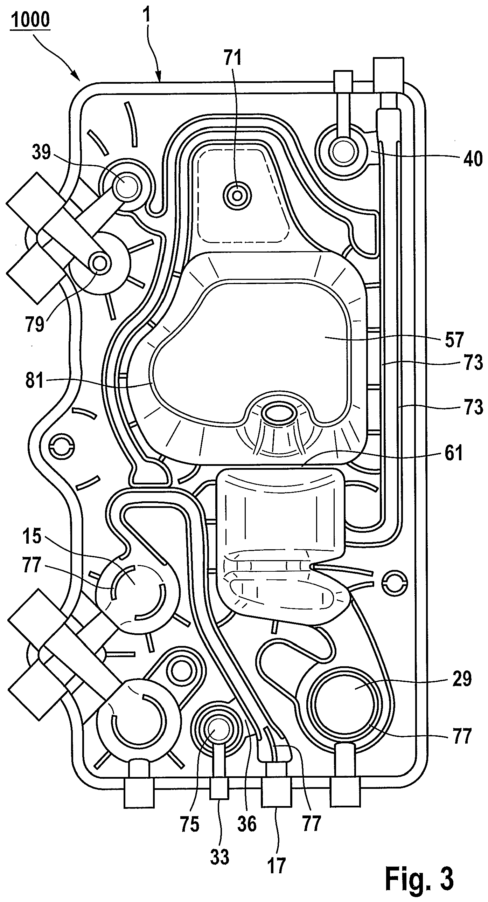

**Fig. 3** zeigt die Kassette 1000 von ihrer Rückseite. Ist die Kassette 1000 an die Blutbehandlungsvorrichtung angekoppelt, so wird ein Betrachter beim Öffnen einer Tür der Blutbehandlungsvorrichtung zum Entnehmen der Kassette 1000 auf diese Rückseite blicken.

Die Kassette 1000 weist einen Single-Needle-Luft-Konnektor 71 auf. Es kann vorgesehen sein, geräteseitig und/oder blutseitig ein Abstützgitter (nicht gezeigt) der Single-Needle-Sterilmembran 55 am Single-Needle-Luft-Konnektor 71 anzuordnen.

Die Kassette 1000 weist mehrere Stützstege auf. Die Stützstege weisen von einander verschiedene Höhen, bezogen beispielsweise auf die Ebene der Folie 3, auf. Die Stützstege erheben sich in der dem Betrachter in Fig. 3 zugewandten Seite der Kassette 1000, also aus der Zeichenebene der Fig. 3 heraus.

Die Kassette 1000 weist Stützstege 73 mit einer Höhe von 5 mm, Stützstege 75 mit einer Höhe von 8 mm, Stützstege 77 mit einer Höhe von 13 mm, Stützstege 79 mit einer Höhe von 24 mm und Stützstege 81 mit einer Höhe von 31 mm auf. Die vorstehenden und andere Zahlenwerte sind natürlich rein exemplarisch zu verstehen.

Die Stützstege können dazu dienen, die Kassette im angekoppelten Zustand an eine Blutbehandlungsvorrichtung gegen einen Deckel einer Aufnahmeeinrichtung der Blutbehandlungsvorrichtung zum Aufnehmen der Kassette abzustützen. Beispielhafte Ausführungsformen einer derartigen Ankopplung der Kassette an die Blutbehandlungsvorrichtung sind in der Patentanmeldung 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, angegeben.

In Fig. 3 ist die Kassette 1000 so gezeigt, wie der Anwender/Betrachter nach ihrem Ankoppeln an das Maschineninterface auf sie blickt. Die Neigung der Kassette 1000 zur Maschine ist "nach hinten geneigt" ausgeführt, so dass die obere Kante weiter vom Anwender/Betrachter entfernt ist als die untere Kante.

Die nach oben zeigenden Flächen der venösen Blutkammer 21 und der Single-Needle-Kammer 57 weisen dementsprechend eine Neigung derart auf, dass Luftblasen trotz der Neigung der Kassette 1000 noch zuverlässig an der Innenseite aufsteigen können. Grundsätzlich ist alternativ natürlich auch ein Kassettendesign möglich, das keine Neigung der Kassette vorsieht.

Die folgenden Figuren zeigen Abschnitte einer erfindungsgemäßen Kassette 1000, welche in allen Merkmalen mit jener Kassette 1000 der Fig. 1 bis 3 übereinstimmen kann, aber nicht muss, soweit sie nicht in den im Folgenden erläuterten Ausführungsformen hiervon abweicht. Jedenfalls kann die erfindungsgemäße Kassette 1000 der folgenden Figuren beliebige Merkmale der in Fig. 1 bis Fig. 3 gezeigten Kassette 1000 aufweisen, sofern die jeweilige Merkmalskombination vom Fachmann nicht als technisch unmöglich erkannt wird.

**Fig. 4** zeigt in Draufsicht einen Ausschnitt eines Hartteils 1 einer erfindungsgemäßen Kassette 1000, beispielsweise jener der vorangegangenen Figuren. Dieses hat Kanäle 201, eine Kammer 202, umlaufende ebene Kanalrandstege 204, an welchen die Folie 3 mit dem Hartteil 1 verklebt oder verschweißt ist. Dargestellt ist ferner ein gedellt ausgeführter Abschnitt 226 des Hartteils 1, welcher sich in die Kanalrandstege 204 fortsetzt oder diese unterbricht. Die Folie 3 ist nicht mit dem darunter liegenden Abschnitt 226 des Hartteils 1 verklebt oder verschweißt, was die oben beschriebene Ventilwirkung ermöglicht. Der Abschnitt 226 wird hierin auch als Foliendichtsitz-Steg 226 oder Ventilgrund 226 bezeichnet. Anstelle der gedellten Ausgestaltung, wie sie in Fig. 4 gezeigt ist, könnte der Abschnitt 226 auch eben oder gerade verlaufen.

Das Bezugszeichen 301 bezeichnet einen Höcker, welcher in der Umgebung des Ventilgrunds 226 und, rein optional, im Kanal 201 angeordnet ist. Der kassettenseitig vorgesehene Höcker 301 könnte auch an anderer Stelle der Kassette 1000 vorgesehen sein, etwa in der Kammer 204. Er ist dabei aber in geeignetem Abstand vom offen zu haltenden Ventil anzuordnen.

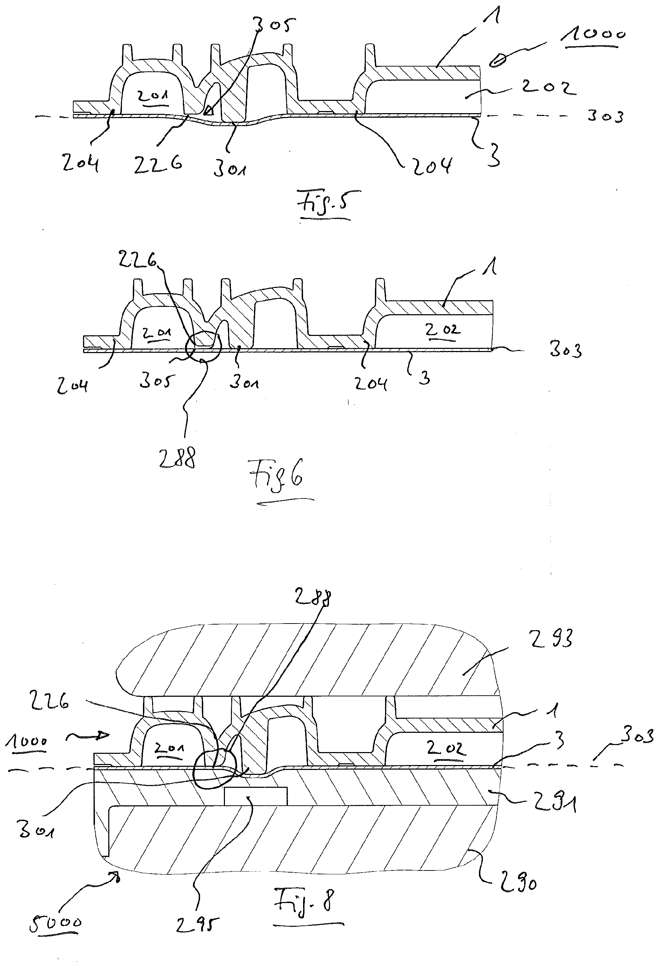

**Fig. 5** zeigt den Blick auf die in Fig. 4 dargestellte Kassette 1000 in einer ersten Ausführungsform in Richtung der Pfeile A der Fig. 4.

Fig. 5 zeigt die Anordnung des Höckers 301 in einer ersten Ausführungsform, in welcher sich dieser über eine Haupterstreckungsebene 303 der Verbindung zwischen Folie 3 und Hartteil 1 hinaus erstreckt oder über diese übersteht. Die (in Fig. 5 gestrichelt dargestellte) Haupterstreckungsebene 303 kann in einigen erfindungsgemäßen Ausführungsformen bei vollkommen ebener Folie 3, wie diese beispielsweise in Fig. 6 gezeigt ist, der Einfachheit halber mit der Ebene, in welcher sich die Folie im Wesentlichen erstreckt, gleichgesetzt werden.

Der Höcker 301 hält die Folie 3 erkennbar auf Abstand zum Ventilgrund 226, was den Spalt 305 ergibt. Letzterer dient der Optimierung der Sterilisation mittels Gases oder Dampfs wie oben diskutiert. Der Ventilgrund 226 kann, wie in Fig. 5 gezeigt, in der Haupterstreckungsebene 303 liegen, so wie auch die Kanalrandstege 204.

**Fig. 6** zeigt den Blick auf die in Fig. 4 dargestellte Kassette 1000 in einer zweiten, möglichen Ausführungsform gemäß der vorliegenden Erfindung, wiederum in Richtung der Pfeile A der Fig. 4.

Fig. 6 zeigt eine Anordnung des Höckers 301, in welcher sich dieser nicht über eine Haupterstreckungsebene 303 der Verbindung zwischen Folie 3 und Hartteil 1 erstreckt oder über diese hinaus übersteht.

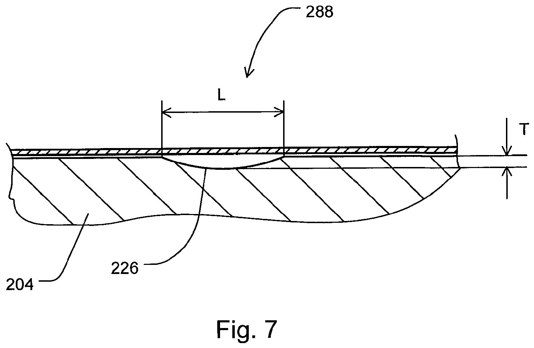

Im dargestellten, exemplarischen Fall der Fig. 6 ist der Abschnitt 226 optional gedellt, oder nicht-gerade, bei einer Dellentiefe T, einer Dellenbreite B und einer Ventilgrund- oder Dichtsitzsteg-Länge L auf, siehe **Fig. 7** zum besseren Verständnis der vorgenannten Dimensionen, wobei sich die nicht darstellbare Dellenbreite B in die Zeichenebene hinein erstreckt.

Die Ausführungsform der Fig. 6 unterscheidet sich von jener der Fig. 5 dadurch, dass der Höcker 301 nicht aus der Haupterstreckungsebene 303 herausragt sondern seine Höckerspitze die Folie 3 berührt. Zudem liegt der Ventilgrund 226 nicht in der Haupterstreckungsebene 303.

Der Höcker 301 hält die Folie 3 gleichwohl erkennbar auf Abstand zum Ventilgrund 226, denn er stellt sicher, dass der Spalt 305 auch im Fall von Unterdruck erhalten bleibt und sich die Folie 3 nicht an den Ventilgrund 226 anlegen kann.

Auch im Beispiel der Fig. 6 hält der Höcker 301 die Folie 3 in geeignetem Abstand zum Grund der Delle, dem Ventilgrund 226.

In der hier beschriebenen erfindungsgemäßen, beispielhaften Ausführungsform beträgt die optimale Breite der Delle zirka das 10- bis 30-fache der Folienstärke und ihre Tiefe zirka das 1- bis 3-fache der Folienstärke.

**Fig. 8** zeigt die Kassette 1000 der Fig. 5, eingelegt in eine Ausführungsform der Blutbehandlungsvorrichtung 5000 und von dieser zwischen Tür 293 und Aktor-Sensor-Matte 291 verpresst. Die Kassette 1000 ist damit im aufgerüsteten, betriebsfertigen Zustand gezeigt, und zwar in Ausschnitten von der Seite mit horizontal ausgerichteter Folienebene oder Haupterstreckungsebene 303.

Die Aktor-Sensor-Matte 291 weist im Bereich des Höckers 301 eine Eindellung oder Aussparung 295 auf. Diese betrifft im hier dargestellten Beispiel nur die von der Blutkassette 1000 abgewandte Seite der Aktor-Sensor-Matte 291. Sie wirkt als Nachgiebigkeits-Zone und ist dergestalt, dass sie ein Schließen des Ventils 288, bei welchem die Folie 3 auch den Ventilsgrund 226 berührt, durch Anpressen der Aktor-Sensor-Matte 291 an die Folie 3 ermöglicht. Eines Aktors zum Schließen des Ventils 288 nach erfolgter Sterilisierung oder vor Beginn der Blutbehandlung bedarf es bei dieser Ausgestaltung vorteilhaft nicht.

Die Aussparung 295 kann, beispielsweise als eine Tasche in der Aktor-Sensor-Matte 291 oder in der Aktor-Sensor-Platte 290 ausgestaltet sein. Dadurch kann die harte Abstützung der Kassette 1000 durch die Aktor-Sensor-Platte 290 lokal aufgehoben sein.

Auch können durch Einfügen definiert nachgiebiger Elemente wie Federn, geschlossenzelliger Schäume, Gummi-Rippen, etc. in die Aussparung oder Tasche Freiräume oder Nachgiebigkeits-Zonen geschaffenen werden. Der Vorteil dieser Ausgestaltung besteht darin, dass sich die in Fig. 8 gezeigt Delle in der Aktor-Sensor-Matte 291 nach dem Abrüsten der Kassette 1000 wieder auflöst, die Aktor-Sensor-Matte 291 also wieder in die ebene Form zurückkehren kann. Dadurch kann die Aktor-Sensor-Matte 291 einfacher und zuverlässiger gereinigt werden. Zudem erlaubt eine solche Aktor-Sensor-Matte 291 eine verbesserte Vakuum-Ankoppelung der Folie 3 von bei nachfolgenden Behandlungen verwendeten Kassetten 1000.

Alternativ kann sich in der Aktor-Sensor-Matte 291 bereits eine lokale, vorgeformte Delle befinden, die in Tiefe und Form einer ausreichend hohen, aber unterhalb der Beschädigungsgrenze liegenden lokalen Eindellung der Folie 3 entspricht. Diese Delle kann optional in beide Richtungen der Folienebene zusätzlich um die mögliche Toleranz der Zuordnung des Höckers 301 zur Delle der Aktor-Sensor-Matte 291 verbreitert sein.

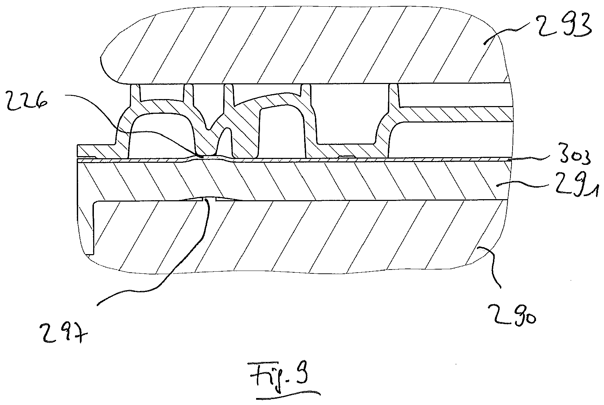

**Fig. 9** zeigt die Kassette 1000 der Fig. 6, eingelegt in eine erfindungsgemäße Ausführungsform der Blutbehandlungsvorrichtung 5000 und von dieser zwischen Tür 293 und Aktor-Sensor-Matte 291 verpresst. Die Kassette 1000 ist damit im aufgerüsteten, betriebsfertigen Zustand gezeigt, und zwar in Ausschnitten, von der Seite, mit horizontal ausgerichteter Folienebene oder Haupterstreckungsebene 303.

Die Aktor-Sensor-Platte 290 weist im Bereich des Ventilgrunds 226 eine Erhebung oder einen Höcker 297 auf. Der Höcker 297 könnte ein integraler Abschnitt der der Aktor-Sensor-Matte 291 sein; im hier dargestellten Beispiel ist der Höcker 297 allerdings angefügte oder integrale Komponente der Aktor-Sensor-Platte 290.

Der Höcker 297 ist in einem dem Ventilgrund 226 der verpressten Kassette 1000 zugeordneten Bereich der Aktor-Sensor-Platte 290 vorgesehen. Er wirkt dergestalt, dass er ein Schließen des Ventils 288, bei welchem die Folie 3 den Ventilsgrund 226 berührt, durch Anpressen der durch den Höcker 297 ausgewölbte oder eingedellte Aktor-Sensor-Matte 291 an die Folie 3 ermöglicht. Eines Aktors zum Schließen des Ventils 288 bedarf es auch bei dieser Ausgestaltung vorteilhaft nicht.

Es sei darauf hingewiesen, dass der Ventilgrund oder der Ventil-Dichtsitz-Steg des Abschnitts 226 keineswegs gedellt sein muss. Auch ein gerader Verlauf des Ventilgrunds 226 ist von der vorliegenden Erfindung umfasst.

Der mit Bezug auf die vorangegangenen Figuren beschriebene, gasdurchlassende Effekt kann erfindungsgemäß auch erzielt werden, wenn ein höherer Höcker oder mehrere nebeneinander parallel zur Ventildichtsitzkante angeordnete Höcker mit entsprechend geringeren Überragungs-Höhendifferenzen vorgesehen sind. In letzterem Fall kann das Folienventil in seiner Erstreckung längs der Kanalkante entsprechend der Anzahl der Höcker länger (bezogen auf seine räumliche Erstreckung) ausgestaltet werden.

Der Höcker 297 kann so gestaltet sein, dass er in Raumrichtung Y (= Vertikale in Figur 4) die Form der Folienventil-Delle im Hartteil 1 mit darüber angeschmiegter Folie 3 abbildet und in Raumrichtung X (= Horizontale in Figur 4) über die Breite des Ventilsteges oder Ventilgrunds 226 plus beidseitiger Positionierungstoleranz diese Form beibehält, um dann verrundet wieder in die Ebene der Aktor-Sensor-Platte 290 auszulaufen. Damit das Ventil 288 durch Schließen und Verpressen der Tür 293 die Folie 3 von der ebenen Initialform in eine angepasste und dicht an die gedellte Ventildichtkante angepresste, verformte Form übergehen kann, ist nicht nur eine ausreichende Nachgiebigkeit der Folie erforderlich, sondern auch eine lokale Verpresskraft. Die allgemeine Verpresskraft der Maschinentür 293 auf die gesamte Kassette 1000 kann bei Kassetten-Versionen mit kanalrandbündigen Verklebungen oder Verschweißungen zu klein gewählt sein, um nach Aufrüstung eine ausreichende Dichtverpressung der Folienventil-Dichtsitzstege gegen die Folie 3 zu gewährleisten. Aus diesem Grund wird vorteilhaft lokal eine erhöhte Verpressung vorgesehen. Diese kann beispielhaft dadurch bewirkt werden, dass die durch den Höcker 297 aufgeworfene Ausbeulung etwas höher, vorzugsweise im Bereich des 1- bis 3-fachen der Folienstärke, ausgestaltet wird. Nach der Verpressung durch die Tür 293 der Blutbehandlungsvorrichtung 5000 baut sich lokal eine höhere Verpressung (mit der Folge der Ventildichtheit) zwischen Folie 3 und Folienventil-Dichtsitzkante 226 auf und das überschüssige Elastomermaterial der Aktor-Sensor-Matte 291 weicht elastisch durch Scherbewegungen in die Bereiche rechts und links der Dichtsitzkante aus. Bei flächiger Auflage der Aktor-Sensor-Matte 291 auf die Aktor-Sensor-Platte 290 ist die Überhöhung der Ausbeulung oder des Höckers 297 derart moderat ausgewählt, dass die elastische Nachgiebigkeit der Aktor-Sensor-Matte 291 ausreicht, um eine vollständige Formanpassung bei gleichmäßiger Verpressung entlang der gedellten Dichtsitzkante zu bewirken, insbesondere, wenn man die Herstellgenauigkeit der Aktor-Sensor-Matte 291 und mögliche geometrische Änderungen durch Abnützungen und Relaxationen berücksichtigt.

Als Ausbeulung oder Höcker 297 kommen erfindungsgemäß auch Vorrichtungen in Frage, welche die Nachgiebigkeit der Aktor-Sensor-Platte 290 und/oder der Aktor-Sensor-Matte 291 lokal erhöhen. Hierzu kommen u. a. Federsysteme in Frage.

**Bezugszeichenliste**

| **Bezugszeichen** | **Beschreibung** |
|---|---|
| 1000 | Kassette |
| 1 | Hartteil |
| 3 | Folie |
| 4 | Dichtsteg |
| 5 | umlaufende Schweißnaht |
| 7 | arterieller Patientenanschluss |
| 9 | arterielle Druckmesskammer |
| 11 | Konnektor für Blutaustritt aus Kassette 1000 |
| 13 | Konnektor für Bluteintritt in Kassette 1000 |
| 15 | Kammer mit arterieller Post-Pumpe- bzw. Präfilter-Druckmessstelle |
| 17 | arterielle Filterleitung |
| 19 | venöse Filterleitung |
| 21 | venöse Blutkammer |
| 23 | oberer Raum der venösen Blutkammer 21 |
| 25 | unterer Raum der venösen Blutkammer 21 |
| 27 | Querschnittsverjüngung des Hartteils 1 |
| 29 | Gerinnselfänger |
| 31 | venöser Patientenanschluss |
| 33 | arterielle Heparin-Zugabestelle |
| 35 | Rückschlagventil der arteriellen Heparin-Zugabestelle 33 |
| 36 | arterielles Heparin-Zugabeventil (Phantomventil) |
| 37 | venöse Heparin-Zugabestelle |
| 39 | Rückschlagventil der venösen Heparin-Zugabestelle 37 |
| 40 | venöses Heparin-Zugabeventil (Phantomventil) |
| 41 | Substituat-Zugabestelle |
| 43 | Konnektor für Substituataustritt aus der Kassette 1000 |
| 45 | Konnektor für Substituateintritt in die Kassette 1000 |
| 47 | Rückschlagventil für Substituatzugabe |
| 49 | Substituatleitung |
| 51 | Prädilutions-Zugabeventil (Phantomventil) |
| 53 | Postdilutions-Zugabeventil (Phantomventil) |
| 55 | Single-Needle-Sterilmembran |
| 57 | Single-Needle-Kammer |
| 59 | Blutschwallumleitungselement |
| 61 | Single-Needle-Blutventil (Phantomventil) |
| 63 | Absaugstelle für Vakuumankopplung |
| 65 | primäres Ausrichtungszentrum |
| 67 | sekundäre Ausrichtungsstelle |
| 69 | Dichtsteg |
| 71 | Single-Needle-Luft-Konnektor |
| 73 | Stützstege mit 5 mm Höhe |
| 75 | Stützstege mit 8 mm Höhe |
| 77 | Stützstege mit 13 mm Höhe |
| 79 | Stützstege mit 24 mm Höhe |
| 81 | Stützstege mit 31 mm Höhe |
| 201 | Kanal |
| 202 | Kammer |
| 204 | umlaufende ebene Kanalrandstege |
| 226 | Abschnitt oder Foliendichtsitz-Steg oder Ventilgrund |
| 288 | Phantom- oder Folienventil |
| 290 | Aktor-Sensor-Platte |
| 291 | Aktor-Sensor-Matte |
| 293 | Tür |
| 295 | Delle oder Aussparung |
| 297 | Höcker oder Erhebung |
| 301 | Höcker |
| 303 | Haupterstreckungsebene |
| 305 | Spalt |
| 5000 | Blutbehandlungsvorrichtung |
| T | Dellentiefe |
| B | Dellenbreite |
| L | Ventilgrund- oder Dichtsitzsteg-Länge |

## Patentansprüche

1. Blutbehandlungskassette (1000) mit einem als Hartteil (1) ausgestalteten Kassettenkörper und einer Folie (3), wobei die Folie (3) mit dem Hartteil (1) verbunden ist und das Hartteil (1) zumindest abschnittsweise abdeckt,
wobei das Hartteil (1) wenigstens einen Ventilgrund (226) eines Ventils (288) aufweist, wobei das Ventil (288) ausgestaltet ist, um neben einer ersten, geöffneten Stellung des Ventils (288), in welcher der Ventilgrund (226) und ein über diesem angeordneter Abschnitt der Folie (3) einander nicht berühren, bei Aufbringen einer Kraft auf einen Abschnitt der Folie (3) eine zweite, geschlossene Stellung des Ventils (288) einzunehmen, in welcher der Ventilgrund (226) und der Abschnitt der Folie (3) einander berühren,
wobei der Ventilgrund (226) in Längsrichtung hiervon konkav oder konvex ausgestaltet ist,
wobei das Hartteil (1) und die Folie (3) sich in einer Hauptberührungsebene (303) berühren und miteinander in der Hauptberührungsebene (303) entlang von Kanalstegen (204), welche Kammern (202) oder Kanäle (201) des Hartteils (1) säumen oder begrenzen, verbunden sind, **dadurch gekennzeichnet dass** die Blutbehandlungskassette (1000) zusätzlich im Bereich des Ventils (288) wenigstens einen mit dem Hartteil (1) integral hergestellten Höcker (301) aufweist,
wobei der Höcker (301) angeordnet ist, um im Nichtgebrauchszustand der Blutbehandlungskassette (1000) ein Verbleiben des Ventils (288) in der ersten, geöffneten Stellung zu gewährleisten

2. Blutbehandlungskassette (1000) nach Anspruch 1, wobei das Ventil (288) ausgestaltet ist, um mittels Druckaufbringung eines Aktuators einer Blutbehandlungsvorrichtung (5000) auf das Ventil (288), zu deren Betrieb die Blutbehandlungskassette (1000) bestimmungsgemäß mit der Blutbehandlungsvorrichtung (5000) verbunden wird, von der ersten Stellung in die zweite Stellung überführbar zu sein.

3. Blutbehandlungskassette (1000) nach Anspruch 1 oder 2, wobei das Ventil (288) als Folien- oder Phantomventil ausgestaltet ist.

4. Blutbehandlungskassette (1000) nach einem der vorangegangenen Ansprüche, wobei der Höcker (301) über die Hauptberührungsebene (303) hinaus in Richtung weg vom Hartteil (1) übersteht.

5. Blutbehandlungskassette (1000) nach Anspruch 4, wobei der Höcker (301) um das 2- bis 4-fache der Dicke der Folie (3) über den Ventilgrund (226) hinausragt.

6. Blutbehandlungskassette (1000) nach einem der vorangegangenen Ansprüche, wobei zwischen dem Ventilgrund (226) und dem nächstgelegenen Abschnitt des Höckers (301) in einer Ebene parallel zur Haupterstreckungsebene (303) der Folie (3) ein Abstand zwischen dem 6- bis 12-fachen der Dicke oder Stärke der Folie (3) vorgesehen ist.

7. Blutbehandlungskassette (1000) nach einem der vorangegangenen Ansprüche, wobei ein Abschnitt des Höckers (301) einen Durchmesser aufweist, welcher das 5-bis 12-fache der Dicke oder Stärke der Folie (3) beträgt.

8. Blutbehandlungskassette (1000) nach einem der vorangegangenen Ansprüche, wobei der Höcker (301) kein Abschnitt der Kanalstege (204) ist, welche Kammern (202) oder Kanäle (201) des Hartteils (1) säumen oder begrenzen.

9. Blutbehandlungskassette (1000) nach einem der vorangegangenen Ansprüche, wobei zwischen dem Höcker (301) und benachbarten Kanalstegen (204) ein Abstand vorgesehen ist, welcher dem 3- bis 8-fachen des Durchmessers des Höckers (301), insbesondere in einem Bereich, in dem der Höcker (301) die Folie (3) berührt, entspricht.

10. Blutbehandlungskassette (1000) nach einem der vorangegangenen Ansprüche, wobei der Ventilgrund (226) auf Höhe der Haupterstreckungsebene (303) der Folie (3) angeordnet ist.

11. Blutbehandlungskassette (1000) nach Anspruch 10, wobei eine Dellentiefe (T) das 2- bis 4-fache der Dicke oder Stärke der Folie (3) beträgt.

12. Blutbehandlungskassette (1000) nach einem der vorangegangenen Ansprüche, wobei der Ventilgrund (226) eine Länge aufweist, welche dem 10- bis 30-fachen der Dicke oder Stärke der Folie (3) entspricht.

13. Blutbehandlungskassette (1000) nach einem der vorangegangenen Ansprüche, wobei eine Dellentiefe (T) des Ventilgrunds (226) dem 1- bis 3-fachen der Dicke oder Stärke der Folie (3) entspricht oder der Ventilgrund (226) hinter benachbarten Kanalrandstegen (204) um das 1- bis 3-fache der Dicke oder Stärke der Folie (3) in Richtung Inneres der Kassette (1000) zurückgesetzt ist.

14. Blutbehandlungsvorrichtung (5000), verbunden mit einer Blutbehandlungskassette (1000) nach einem der Ansprüche 1 bis 13, mit einer Aktor-Sensor-Platte (290) mit wenigstens einem Aktuator für eine Interaktion zwischen Aktoren und/oder Sensoren der Blutbehandlungsvorrichtung (5000) mit Vorrichtungen der Blutbehandlungskassette (1000).

15. Blutbehandlungsvorrichtung (5000) nach Anspruch 14, wobei die Aktor-Sensor-Platte (290) eine Aktor-Sensor-Matte (291) aufweist, welche in Kontakt mit der Blutbehandlungskassette (1000) steht, wobei die Aktor-Sensor-Matte (291) in einem Abschnitt, welcher in Kontakt mit dem Höcker (301) der Blutbehandlungskassette (1000) steht, dünner ist als benachbarte Abschnitte der Aktor-Sensor-Matte (291).

16. Blutbehandlungsvorrichtung (5000) nach Anspruch 15, wobei die Aktor-Sensor-Matte (291) im Abschnitt, welcher in Kontakt mit dem Höcker (301) der Blutbehandlungskassette (1000) steht, eine Delle oder eine Aussparung (295) derart hat, dass sie im Bereich ihrer Delle oder Aussparung nicht mit der Aktor-Sensor-Platte (290) in Berührung steht.

17. Blutbehandlungsvorrichtung (5000) nach einem der Ansprüche 14 bis 16, wobei die Aktor-Sensor-Platte (290) eine Aktor-Sensor-Matte (291) aufweist, welche in Kontakt mit der Blutbehandlungskassette (1000) steht, wobei die Aktor-Sensor-Platte (290) und/oder die Aktor-Sensor-Matte (291) in einem Abschnitt, welcher in Kontakt mit dem Ventilgrund (226) des Ventils (288) steht, einen Höcker (297) oder eine Verstärkung aufweist.

## Claims

1. A blood treatment cassette (1000) having a cassette body designed as a hard part (1) and a film (3), the film (3) being connected to the hard part (1) and covering the hard part (1) at least in portions,
wherein the hard part (1) comprises at least one valve base (226) of a valve (288), the valve (288) being designed so that it takes, when applying a force to a portion of the film (3), in addition to a first open position of the valve (288) in which the valve base (226) and a portion of the film (3) covering it don't touch each other,
a second closed position of the valve (288) in which the valve base (226) and the portion of the film (3) touch each other,
wherein the valve base (226) is designed concave or convex in its longitudinal direction,
wherein the hard part (1) and the film (3) touch each other in a main contact plane (303) and are connected to each other in the main contact plane (303) along channel bridges (204), which border or delimit chambers (202) or channels (201) of the hard part (1),
**characterized in that**
the blood treatment cassette (1000) additionally comprises, in a region of the valve (288), at least one hump (301) integrally produced with the hard part (1),
wherein the hump (301) is arranged to ensure that the valve (288) remains in the first open position when the blood treatment cassette (1000) is in a non-use state.

2. The blood treatment cassette (1000) according to claim 1, wherein the valve (288) is designed to be transferable from the first position to the second position by means of pressure application of an actuator of a blood treatment device (5000) to the valve (288), to the operation of which the blood treatment cassette (1000) is connected, as intended, to the blood treatment device (5000).

3. The blood treatment cassette (1000) according to claim 1 or 2, wherein the valve (288) is designed as a film valve or as a phantom valve.

4. The blood treatment cassette (1000) according to anyone of the preceding claims, wherein the hump (301) projects beyond the main contact plane (303) in a direction away from the hard part (1).

5. The blood treatment cassette (1000) according to claim 4, wherein the hump (301) projects 2 to 4 times the thickness of the film (3) beyond the valve base (226).

6. The blood treatment cassette (1000) according to anyone of the preceding claims, wherein between the valve base (226) and the nearest portion of the hump (301) in a plane parallel to the main extension plane (303) of the film (3), a distance between 6 to 12 times the thickness or strength of the film (3) is provided.

7. The blood treatment cassette (1000) according to anyone of the preceding claims, wherein a portion of the hump (301) comprises a diameter amounting 5 to 12 times the thickness or strength of the film (3).

8. The blood treatment cassette (1000) according to anyone of the preceding claims, wherein the hump (301) is not a portion of the channel bridges (204) bordering or delimiting chambers (202) or channels (201) of the hard part (1).

9. The blood treatment cassette (1000) according to anyone of the preceding claims, wherein between the hump (301) and adjacent channel bridges (204), in particular in a region where the hump (301) touches the film (3), a distance corresponding to 3 to 8 times the diameter of the hump (301) is provided.

10. The blood treatment cassette (1000) according to anyone of the preceding claims, wherein the valve base (226) is arranged at the level of the main extension plane (303) of the film (3) .

11. The blood treatment cassette (1000) according to claim 10, wherein a depression depth (T) amounts 2 to 4 times the thickness or strength of the film (3).

12. The blood treatment cassette (1000) according to anyone of the preceding claims, wherein the valve base (226) has a length corresponding to 10 to 30 times the thickness or strength of the film (3).

13. The blood treatment cassette (1000) according to anyone of the preceding claims, wherein a depression depth (T) of the valve base (226) corresponds to 1 to 3 times the thickness or strength of the film (3) or wherein the valve base (226) is set back, behind adjacent channel border bridges (204), 1 to 3 times the thickness or strength of the film (3) towards the inside of the cassette (1000).

14. A blood treatment apparatus (5000) connected with a blood treatment cassette (1000) according to anyone of claims 1 to 13, comprising an actuator sensor plate (290) having at least one actuator for interaction between actuators and/or sensors of the blood treatment apparatus (5000) with apparatuses of the blood treatment cassette (1000).

15. The blood treatment apparatus (5000) according to claim 14, wherein the actuator sensor plate (290) comprises an actuator sensor mat (291), which is in contact with the blood treatment cassette (1000), wherein the actuator sensor mat (291) is thinner than adjacent portions of the actuator sensor mat (291) in a portion being in contact with the hump (301) of the blood treatment cassette (1000).

16. The blood treatment apparatus (5000) according to claim 15, wherein the actuator sensor mat (291) comprises a depression or recess (295) in the portion in contact with the hump (301) of the blood treatment cassette (1000), so that it doesn't touch the actuator sensor plate (290) in the region of its depression or recess.

17. The blood treatment apparatus (5000) according to anyone of claims 14 to 16, wherein the actuator sensor plate (290) comprises an actuator sensor mat (291), which is in contact with the blood treatment cassette (1000), wherein the actuator sensor plate (290) and/or the actuator sensor mat (291) comprise(s) a hump (297) or a reinforcement in a portion, which is in contact with the valve base (226) of the valve (288).

## Revendications

1. Une cassette de traitement du sang (1000) ayant un corps de cassette conçu comme une partie rigide (1) et un film (3), le film (3) étant relié à la partie rigide (1) et recouvrant la partie rigide (1) au moins par sections,
où la partie rigide (1) comprend au moins un fond de vanne (226) d'une vanne (288), la vanne (288) étant conçue de manière à ce que, lors de l'application d'une force sur une section du film (3), elle prenne, en plus d'une première position ouverte de la vanne (288) dans laquelle le fond de vanne (226) et une section du film (3) qui la recouvre ne se touchent pas,
une seconde position fermée de la vanne (288) dans laquelle le fond de vanne (226) et la section du film (3) se touchent,
où le fond de vanne (226) est conçu de manière concave ou convexe dans sa direction longitudinale,
où la partie rigide (1) et le film (3) se touchent dans un plan de contact principal (303) et sont reliés l'un à l'autre dans le plan de contact principal (303) le long de traverses de conduits (204) bordant ou délimitant des chambres (202) ou des conduits (201) de la partie rigide (1),
**caractérisée en ce que**
la cassette de traitement du sang (1000) comprend en outre, dans la zone de la vanne (288), au moins un renflement (301) réalisé de façon intégrale avec la partie rigide (1),
où le renflement (301) est agencé de manière à assurer le maintien de la vanne (288) dans la première position ouverte lorsque la cassette de traitement du sang (1000) est en état de non-utilisation.

2. La cassette de traitement du sang (1000) selon la première revendication, où la vanne (288) est conçue pour être transférable de la première à la seconde position par application d'une pression d'un actionneur d'un appareil de traitement du sang (5000) sur la vanne (288), pour le fonctionnement de laquelle la cassette de traitement du sang (1000) est reliée, conformément à son usage prévu, à l'appareil de traitement du sang (5000).

3. La cassette de traitement du sang (1000) selon la revendication 1 ou 2, où la vanne (288) est conçue comme une vanne à membrane ou une vanne fantôme.

4. La cassette de traitement du sang (1000) selon l'une quelconque des revendications précédentes, où le renflement (301) fait saillie au-delà du plan de contact principal (303), dans une direction s'éloignant de la partie rigide (1).

5. La cassette de traitement du sang (1000) selon la revendication 4, où le renflement (301) fait saillie de 2 à 4 fois l'épaisseur du film (3) au-dessus du fond de vanne (226).

6. La cassette de traitement du sang (1000) selon l'une quelconque des revendications précédentes, où entre le fond de vanne (226) et la section la plus proche du renflement (301), dans un plan parallèle au plan d'extension principal (303) du film (3), une distance comprise entre 6 et 12 fois l'épaisseur ou la hauteur du film (3) est prévue.

7. La cassette de traitement du sang (1000) selon l'une quelconque des revendications précédentes, où une section du renflement (301) présente un diamètre s'élevant à 5 à 12 fois l'épaisseur ou la hauteur du film (3).

8. La cassette de traitement du sang (1000) selon l'une quelconque des revendications précédentes, où le renflement (301) n'est pas une section des traverses de conduits (204) bordant ou délimitant des chambres (202) ou des conduits (201) de la partie rigide (1).

9. La cassette de traitement du sang (1000) selon l'une quelconque des revendications précédentes, où une distance correspondant à 3 à 8 fois le diamètre du renflement (301) est prévue entre le renflement (301) et les traverses de conduits (204) adjacentes, en particulier dans une zone où le renflement (301) touche le film (3).

10. La cassette de traitement du sang (1000) selon l'une quelconque des revendications précédentes, où le fond de vanne (226) est agencé au niveau du plan d'extension principal (303) du film (3).

11. La cassette de traitement du sang (1000) selon la revendication 10, où une profondeur d'enfoncement (T) s'élève à 2 à 4 fois l'épaisseur ou la hauteur du film (3) .

12. La cassette de traitement du sang (1000) selon l'une quelconque des revendications précédentes, où le fond de vanne (226) présente une longueur correspondant à 10 à 30 fois l'épaisseur ou la hauteur du film (3).

13. La cassette de traitement du sang (1000) selon l'une quelconque des revendications précédentes, où une profondeur d'enfoncement (T) du fond de vanne (226) correspond à 1 à 3 fois l'épaisseur ou la hauteur du film (3), ou où le fond de vanne (226) est décalé à l'arrière de bords de traverses de conduits (204) adjacents de 1 à 3 fois l'épaisseur ou la hauteur du film (3) en direction de l'intérieur de la cassette (1000).

14. Un appareil de traitement du sang (5000) relié à une cassette de traitement du sang (1000) selon l'une quelconque des revendications 1 à 13, comprenant une plaque actionneur-capteur (290) ayant au moins un actionneur pour une interaction entre des actionneurs et/ou des capteurs de l'appareil de traitement du sang (5000) avec des appareils de la cassette de traitement du sang (1000).

15. L'appareil de traitement du sang (5000) selon la revendication 14, où la plaque actionneur-capteur (290) comprend un tapis actionneur-capteur (291) en contact avec la cassette de traitement du sang (1000), où, dans une section étant en contact avec le renflement (301) de la cassette de traitement du sang (1000), le tapis actionneur-capteur (291) est plus fin que les sections adjacentes du tapis actionneur-capteur (291).

16. L'appareil de traitement du sang (5000) selon la revendication 15, où le tapis actionneur-capteur (291) comprend un enfoncement (295) ou un évidement dans la section étant en contact avec le renflement (301) de la cassette de traitement du sang (1000), de sorte qu'il ne touche pas la plaque actionneur-capteur (290) dans la zone de son enfoncement ou de son évidement.

17. L'appareil de traitement du sang (5000) selon l'une quelconque des revendications 14 à 16, où la plaque actionneur-capteur (290) comprend un tapis actionneur-capteur (291) en contact avec la cassette de traitement du sang (1000), où la plaque actionneur-capteur (290) et/ou le tapis actionneur-capteur (291) comprend/comprennent un renflement (297) ou un renforcement dans une section en contact avec le fond de vanne (226) de la vanne (288).
